# EUROPÄISCHE PATENTSCHRIFT

(11) **EP 3 799 243 B1**
(45) Veröffentlichungstag und Bekanntmachung des Hinweises auf die Patenterteilung: **28.06.2023**
(21) Anmeldenummer: 19199612.3
(22) Anmeldetag: 25.09.2019
(51) Int. Cl.: H02J 1/12, A61B 6/00, H02J 5/00, H02J 7/34

(54) **COMPUTERTOMOGRAPHIEGERÄT UND VERFAHREN ZUR LEISTUNGSVERSORGUNG EINES RÖNTGENSYSTEMS EINES COMPUTERTOMOGRAPHIEGERÄTS**
COMPUTER TOMOGRAPHY APPARATUS AND METHOD FOR SUPPLYING POWER TO AN X-RAY SYSTEM OF A COMPUTER TOMOGRAPHY APPARATUS
APPAREIL DE TOMODENSITOMÉTRIE ET PROCÉDÉ D'ALIMENTATION EN PUISSANCE D'UN SYSTÈME DE RAYONS X D'UN APPAREIL DE TOMODENSITOMÉTRIE

(43) Veröffentlichungstag der Anmeldung: 31.03.2021
(73) Patentinhaber: Siemens Healthcare GmbH, 91052 Erlangen (DE)
(72) Erfinder: Gebert, Bernhard, 91083 Hagenau (DE); Krauss, Markus, 91344 Waischenfeld (DE)

(56) Entgegenhaltungen:
- DE-A1- 19 633 359
- DE-A1-102010 042 565
- US-A1- 2008 112 537
- US-A1- 2017 027 537
- US-A1- 2017 085 122
- US-A1- 2017 285 119

## Beschreibung

Die Erfindung betrifft ein Computertomographiegerät und ein Verfahren zur Leistungsversorgung eines Röntgensystems eines Computertomographiegeräts.

Die Eingangsleistung von Computertomographiegeräten mit zwei Röntgenstrahlern (Zweistrahlersystem) liegt typischerweise bei mehreren 100 kVA, was zu sehr hohen Anforderungen an den Netzanschluss und die Gebäudeinstallation, insbesondere an die Zuleitungen, den Netzinnenwiderstand und die Sicherungswerte, führt. Tatsächlich wird diese maximale Leistung aber nur während weniger Scanmodes erreicht und dort nur für sehr kurze Zeit.

Die mittlere Leistungsaufnahme eines Zweistrahlersystems liegt daher geringfügig über dem Maximalwert der Leistungsaufnahme eines Computertomographiegeräts mit nur einem Röntgenstrahler (Einstrahlersystem). Durch einen nicht idealen 90°-Versatz der beiden Röntgenstrahler, der langsamen Abkühlzeit des Emitters und ungünstiger Überlappungen der Dosisverläufe, beispielsweise im Bereich der Schulter und des Halses, treten kürzere Lastspitzen auf, die gepuffert werden können. Einstrahlersysteme verhalten sich in Bezug auf die Leistungsaufnahme ähnlich, hier liegt der Mittelwert der Aufnahmeleistung meist bei unter 50 % des Spitzenwerts.

Bisher gibt es die Möglichkeit, das Computertomographiegerät mit einer unterbrechungsfreien Stromversorgung zu betreiben, die zur Pufferung der Lastspitzen genutzt wird. Diese Möglichkeit ist sehr kostenintensiv und benötigt viel Bauraum. Außerdem gibt es Einrichtungen zur aktiven Nachregelung der Netzspannung, mit denen ein vorhandener Netzanschluss besser ausgenutzt werden kann, was unter Umständen zu Komplikationen im praktischen Betrieb führen kann.

Zweistrahlersysteme können beispielsweise mit zwei separaten Netzanschlussleitungen versorgt werden. Das kann jedoch zu erheblichen Kosten bei dem Netzeingang und dem Schleifring, der die Energie zum rotierenden Teil überträgt, führen.

DE 10 2010 042565 A1 offenbart eine Vorrichtung zur Versorgung eines bildgebenden medizinischen Gerätes mit elektrischer Energie.

US 2017/027537 A1 offenbart ein Stromversorgungsgerät für ein medizinisch-diagnostisches Hochfrequenz-Röntgengerät.

US 2017/285119 A1 offenbart ein Verfahren zum Umgang mit einem Spitzenleistungsbedarf eines medizinischen Bildgebungsgeräts.

US 2017/085122 A1 offenbart eine unterbrechungsfreie Stromversorgung mit Lastausgleich für medizinische Bildgebungslasten.

US 2008/112537 A1 offenbart ein Computertomographiesystem mit einer Speichervorrichtung, die konfiguriert ist, um die Leistungsabgabe mit einer Eingangsstromleitung zu teilen, um Spitzenlastanforderungen der Eingangsstromleitung zu reduzieren.

DE 196 33 359 A1 offenbart eine Zweiebenen-Röntgendiagnostikanlage mit einem Hochfrequenz-Röntgengenerator.

Die Erfindung hat die Aufgabe, eine Alternative zu einer herkömmlichen Leistungsversorgung eines Röntgensystems eines Computertomographiegeräts mit zwei Röntgenstrahlern bereitzustellen. Jeder Gegenstand eines unabhängigen Anspruchs löst diese Aufgabe. In den abhängigen Ansprüchen sind weitere vorteilhafte Aspekte der Erfindung berücksichtigt.

Die Erfindung betrifft ein Computertomographiegerät, aufweisend
- ein Röntgensystem und einen Zwischenkreis, der auf Gleichstrom basiert, wobei das Röntgensystem zur Entnahme einer resultierenden Leistung aus dem Zwischenkreis ausgebildet ist,
- einen ersten Pfad, der zur Entnahme einer Leistung des ersten Pfads aus einem Niederspannungsnetz und zum Einspeisen der Leistung des ersten Pfads in den Zwischenkreis ausgebildet ist, wobei der erste Pfad einen Gleichrichter zur Verbindung des Zwischenkreises mit dem Niederspannungsnetz aufweist,
- einen zweiten Pfad, der zur Entnahme einer elektrischen Energie aus dem Niederspannungsnetz und zum Einspeisen einer Leistung des zweiten Pfads in den Zwischenkreis basierend auf der elektrischen Energie ausgebildet ist, um durch die Leistung des zweiten Pfads eine Differenz zwischen der resultierenden Leistung und der Leistung des ersten Pfads auszugleichen, wobei der zweite Pfad einen Energiespeicher zum Speichern der Energie, der auf einer elektrischen Kapazität und/oder auf einer elektrochemischen Pseudokapazität basiert, ein Eingangsmodul zur Verbindung des Energiespeichers mit dem Niederspannungsnetz und ein Ausgangsmodul zur Verbindung des Energiespeichers mit dem Zwischenkreis aufweist.

Insbesondere kann der Energiespeicher zum Speichern der elektrischen Energie, insbesondere zum Speichern der elektrischen Energie nach der Entnahme der elektrischen Energie aus dem Niederspannungsnetz und/oder vor dem Einspeisen der Leistung des zweiten Pfads in den Zwischenkreis basierend auf der elektrischen Energie, ausgebildet sein.

Das Niederspannungsnetz kann beispielsweise auf Wechselstrom und/oder einer Netzspannung von weniger als 1000 Volt basieren. Im Kontext dieser Anmeldung wird unter Leistung stets eine elektrische Leistung verstanden.

Das Eingangsmodul kann insbesondere zum Laden der elektrischen Energie aus dem Niederspannungsnetz in den Energiespeicher ausgebildet sein. Eine Ausführungsform sieht vor, dass das Eingangsmodul einen Netzgleichrichter und eine Ladeschaltung aufweist, dass die Ladeschaltung mittels des Netzgleichrichters mit dem Niederspannungsnetz verbunden ist und dass der Energiespeicher mittels der Ladeschaltung mit dem Netzgleichrichter verbunden ist.

Insbesondere kann mittels des Netzgleichrichters die elektrische Energie aus dem Niederspannungsnetz in Form eines Netz-Wechselstromes entnommen und in Form eines Lade-Gleichstroms in die Ladeschaltung eingespeist werden. Mittels der Ladeschaltung kann die elektrische Energie in den Energiespeicher geladen werden, indem die elektrische Kapazität und/oder die elektrochemische Pseudokapazität aufgeladen wird.

Eine Ausführungsform sieht vor, dass das Eingangsmodul einen Leistungsfaktorkorrekturfilter (englisch Power Factor Correction) aufweist. Damit können Störungen des Niederspannungsnetzes durch das Aufladen des Energiespeichers weitgehend vermieden werden. Ein Aufladen des Energiespeichers auf eine relativ hohe Spannung ist insbesondere für Scans sinnvoll, bei denen über einen längeren Zeitabschnitt eine konstant hohe resultierende Leistung entnommen wird, beispielsweise bei einem Cardio-Scan mit einem Zweistrahlersystem. Das Aufladen des Energiespeichers kann dann insbesondere vor Beginn des Scans erfolgen, um eine hohe Belastung des Niederspannungsnetzes während des Scans zu vermeiden.

Insbesondere kann der Energiespeicher auf eine deutlich höhere Spannung als eine Zwischenkreisspannung des Zwischenkreises aufgeladen und/oder als Stromquelle zum Nachladen des Zwischenkreises verwendet werden. Insbesondere kann vorgesehen sein, dass ein maximaler Wert der Leistung des ersten Pfads kleiner als ein maximaler Wert der resultierenden Leistung ist.

Das Ausgangsmodul kann insbesondere zum Einspeisen der Leistung des zweiten Pfads basierend auf der elektrischen Energie aus dem Energiespeicher in den Zwischenkreis ausgebildet sein. Eine Ausführungsform sieht vor, dass das Ausgangsmodul eine Schutzschaltung aufweist und dass der Zwischenkreis mittels der Schutzschaltung mit dem Energiespeicher verbunden ist.

Eine Ausführungsform sieht vor, dass das Ausgangsmodul einen Wechselrichter und einen Gleichrichter zur Verbindung des Zwischenkreises mit dem Wechselrichter aufweist und dass der Zwischenkreis mittels des Wechselrichters und des Gleichrichters zur Verbindung des Zwischenkreises mit dem Wechselrichter mit dem Energiespeicher verbunden ist.

Insbesondere kann der Wechselrichter dazu ausgebildet sein, ein Entladen des Energiespeichers zu unterstützen, beispielsweise ein tieferes Entladen des Energiespeichers zu ermöglichen. Dieser Wechselrichter kann für eine relativ hohe Leistung, die jedoch nur während relativ kurzer Zeitabschnitte vorübergehend auftritt, ausgelegt sein.

Eine Ausführungsform sieht vor, dass das Computertomographiegerät einen Tragrahmen und einen Rotor, der relativ zu dem Tragrahmen um eine Rotationsachse drehbar gelagert ist, aufweist, wobei der Rotor das Röntgensystem und den Zwischenkreis aufweist. Das Computertomographiegerät kann insbesondere eine Drehübertragungsvorrichtung aufweisen, die zur Drehübertragung der Leistung des ersten Pfads ausgebildet ist und die ferner zur Drehübertragung der elektrischen Energie oder der Leistung des zweiten Pfads ausgebildet ist. Insbesondere kann vorgesehen sein, dass der erste Pfad und der zweite Pfad einen gemeinsamen Abschnitt aufweisen. Der gemeinsame Abschnitt kann beispielsweise unmittelbar an das Niederspannungsnetz anschließen und/oder die Drehübertragungsvorrichtung aufweisen. In dem gemeinsamen Abschnitt können beispielsweise die Leistung des ersten Pfads und die elektrische Energie des zweiten Pfads gleichzeitig mittels desselben elektrischen Leiters übertragen werden.

Insbesondere dann, wenn der Energiespeicher relativ zu dem Tragrahmen fest angeordnet ist, kann der Wechselrichter mittels der Drehübertragungsvorrichtung mit dem Gleichrichter zur Verbindung des Zwischenkreises mit dem Wechselrichter verbunden sein.

Der erste Pfad ist zur durchgehenden Übertragung der Leistung des ersten Pfads von dem Niederspannungsnetz bis zu dem Zwischenkreis ausgebildet. Insbesondere ist eine statische Speicherung elektrischer Energie auf dem ersten Pfad nicht vorgesehen.

Erfindungsgemäß ist vorgesehen, dass das Röntgensystem einen ersten Röntgenstrahler und einen zweiten Röntgenstrahler aufweist. In diesem Fall kann das Computertomographiegerät als Zweistrahlersystem bezeichnet werden. Eine weitere Ausführungsform sieht vor, dass das Röntgensystem mehr als zwei Röntgenstrahler aufweist.

Insbesondere kann der erste Röntgenstrahler zum Erzeugen einer ersten Röntgenstrahlung, die auf einen ersten Detektor des Computertomographiegeräts ausgerichtet ist, basierend auf der Leistung des ersten Röntgenstrahlers ausgebildet sein. Dabei kann ein Teil der Leistung des ersten Röntgenstrahlers in Wärme des ersten Röntgenstrahlers umgewandelt werden. Insbesondere kann der zweite Röntgenstrahler zum Erzeugen einer zweiten Röntgenstrahlung, die auf einen zweiten Detektor des Computertomographiegeräts ausgerichtet ist, basierend auf der Leistung des zweiten Röntgenstrahlers ausgebildet sein. Dabei kann ein Teil der Leistung des zweiten Röntgenstrahlers in Wärme des zweiten Röntgenstrahlers umgewandelt werden.

Der erste Röntgenstrahler ist zur Entnahme einer Leistung des ersten Röntgenstrahlers aus dem Zwischenkreis ausgebildet. Der zweite Röntgenstrahler ist zur Entnahme einer Leistung des zweiten Röntgenstrahlers aus dem Zwischenkreis ausgebildet. Erfindungsgemäß ist vorgesehen, dass die resultierende Leistung aus der Leistung des ersten Röntgenstrahlers und der Leistung des zweiten Röntgenstrahlers resultiert, beispielsweise dass die resultierende Leistung eine Summe der Leistung des ersten Röntgenstrahlers und der Leistung des zweiten Röntgenstrahlers ist.

Insbesondere kann der erste Röntgenstrahler zur Entnahme der Leistung des ersten Röntgenstrahlers aus dem Zwischenkreis in Form eines ersten Gleichstroms ausgebildet sein. Insbesondere kann der erste Röntgenstrahler einen ersten Hochspannungsgenerator zur Erzeugung einer ersten Hochspannung basierend auf dem ersten Gleichstrom aufweisen. Insbesondere kann der zweite Röntgenstrahler zur Entnahme der Leistung des zweiten Röntgenstrahlers aus dem Zwischenkreis in Form eines zweiten Gleichstroms ausgebildet sein. Insbesondere kann der zweite Röntgenstrahler einen zweiten Hochspannungsgenerator zur Erzeugung einer zweiten Hochspannung basierend auf dem zweiten Gleichstrom aufweisen.

Erfindungsgemäß ist vorgesehen, dass in einem ersten Betriebszustand des Computertomographiegeräts ein Zeitverlauf der resultierenden Leistung periodisch ist, ein Zeitverlauf der Leistung des ersten Pfads konstant ist und ein Zeitverlauf der Leistung des zweiten Pfads periodisch ist.

Erfindungsgemäß ist vorgesehen, dass der zweite Pfad derart ausgebildet ist, dass in dem ersten Betriebszustand des Computertomographiegeräts die elektrische Energie aus dem Wechselstromkreis in den Energiespeicher geladen werden kann, während der Zeitverlauf der resultierenden Leistung einen minimalen Wert der resultierenden Leistung durchläuft, und die Leistung des zweiten Pfads basierend auf der elektrischen Energie aus dem Energiespeicher in den Zwischenkreis eingespeist werden kann, während der Zeitverlauf der resultierenden Leistung einen maximalen Wert der resultierenden Leistung durchläuft.

Erfindungsgemäß ist vorgesehen,
- dass das Röntgensystem um eine Rotationsachse drehbar gelagert ist,
- dass der erste Röntgenstrahler und der zweite Röntgenstrahler in einer Rotationsebene, die zu der Rotationsachse senkrecht ist, um einen Systemwinkel, dessen Scheitelpunkt auf der Rotationsachse liegt, zueinander versetzt angeordnet sind,
- dass in dem ersten Betriebszustand des Computertomographiegeräts ein Zeitverlauf der Leistung des ersten Röntgenstrahlers periodisch ist und ein Zeitverlauf der Leistung des zweiten Röntgenstrahlers periodisch ist,
- dass eine Phasenverschiebung zwischen dem Zeitverlauf der Leistung des ersten Röntgenstrahlers und dem Zeitverlauf der Leistung des zweiten Röntgenstrahlers dem Systemwinkel entspricht.

Insbesondere kann in dem ersten Betriebszustand des Computertomographiegeräts eine Drehung des Rotors um die Rotationsachse, um die er drehbar gelagert ist, erfolgen. Beispielsweise kann eine Periodendauer des Zeitverlaufs der Leistung des ersten Röntgenstrahlers gleich einer Dauer einer vollen oder halben Umdrehung des Röntgensystems um die Rotationsachse sein. Eine Periodendauer des Zeitverlaufs der Leistung des ersten Röntgenstrahlers ist gleich einer Periodendauer eines Zeitverlaufs der Leistung des zweiten Röntgenstrahlers.

Beispielweise kann eine Dauer einer vollen Umdrehung des Röntgensystems um die Rotationsachse kleiner als 400 Millisekunden, insbesondere kleiner als 300 Millisekunden, beispielsweise kleiner als 250 Millisekunden sein. Beispielweise kann eine Dauer einer halben Umdrehung des Röntgensystems um die Rotationsachse kleiner als 200 Millisekunden, insbesondere kleiner als 150 Millisekunden, beispielsweise kleiner als 125 Millisekunden sein. Eine Ausführungsform der Erfindung sieht vor, dass die Periodendauer des Zeitverlaufs der Leistung des ersten Röntgenstrahlers größer als 100 Millisekunden ist, insbesondere größer als 125 Millisekunden ist.

Insbesondere können der Zeitverlauf der Leistung des ersten Röntgenstrahlers und der Zeitverlauf der Leistung des zweiten Röntgenstrahlers bis auf die Phasenverschiebung identisch sein. Beispielsweise kann in dem ersten Betriebszustand des Computertomographiegeräts der Zeitverlauf der Leistung des ersten Röntgenstrahlers und/oder der Zeitverlauf der Leistung des zweiten Röntgenstrahlers auf einer Dosismodulation basieren. Der Zeitverlauf der Leistung des ersten Röntgenstrahlers und/oder der Zeitverlauf der Leistung des zweiten Röntgenstrahlers kann beispielsweise gepulst sein oder kontinuierlich oszillierend sein, beispielsweise im Wesentlichen sinusförmig, insbesondere sinusförmig sein.

Der Systemwinkel kann insbesondere im Wesentlichen 90 Grad, beispielsweise 90 Grad oder 95 Grad betragen. Der Systemwinkel kann beispielsweise größer als 80 Grad sein und/oder kleiner als 100 Grad sein. Ein maximaler Wert der Leistung des ersten Röntgenstrahlers erfolgt somit im Wesentlichen zeitgleich mit einem minimalen Wert der Leistung des zweiten Röntgenstrahlers.

Insbesondere kann vorgesehen sein, dass in dem ersten Betriebszustand des Computertomographiegeräts ein maximaler Wert der Leistung des zweiten Pfads kleiner als die Leistung des ersten Pfads ist, beispielsweise kleiner als 50% der Leistung des ersten Pfads ist, insbesondere kleiner als 30% der Leistung des ersten Pfads ist.

Beispielsweise kann in dem ersten Betriebszustand des Computertomographiegeräts der maximale Wert der Leistung des zweiten Pfads kleiner als 70 Kilowatt, insbesondere kleiner als 50 Kilowatt, beispielsweise kleiner als 30 Kilowatt sein. Beispielsweise kann in dem ersten Betriebszustand des Computertomographiegeräts der maximale Wert der Leistung des zweiten Pfads gleich 50 Kilowatt sein.

Beispielsweise kann in dem ersten Betriebszustand des Computertomographiegeräts eine Dauer des Ladens der elektrischen Energie aus dem Niederspannungsnetz in den Energiespeicher, während der Zeitverlauf der resultierenden Leistung einen minimalen Wert der resultierenden Leistung durchläuft, kleiner als 100 Millisekunden, insbesondere kleiner als 70 Millisekunden, beispielsweise kleiner als 40 Millisekunden, beispielsweise gleich 30 Millisekunden sein.

Beispielsweise kann in dem ersten Betriebszustand des Computertomographiegeräts eine Dauer des Einspeisens der Leistung des zweiten Pfads basierend auf der elektrischen Energie aus dem Energiespeicher in den Zwischenkreis, während der Zeitverlauf der resultierenden Leistung einen minimalen Wert der resultierenden Leistung durchläuft, kleiner als 120 Millisekunden, insbesondere kleiner als 90 Millisekunden, beispielsweise kleiner als 60 Millisekunden, beispielsweise gleich 50 Millisekunden, sein.

Beispielsweise kann in dem ersten Betriebszustand des Computertomographiegeräts eine Dauer des Einspeisens der Leistung des zweiten Pfads basierend auf der elektrischen Energie aus dem Energiespeicher in den Zwischenkreis, während der Zeitverlauf der resultierenden Leistung einen minimalen Wert der resultierenden Leistung durchläuft, größer als 10 Millisekunden, insbesondere größer als 20 Millisekunden, beispielsweise größer als 30 Millisekunden, beispielsweise gleich 50 Millisekunden, sein.

Eine Ausführungsform sieht vor, dass in einem zweiten Betriebszustand des Computertomographiegeräts ein Zeitverlauf der resultierenden Leistung konstant ist, ein Zeitverlauf der Leistung des ersten Pfads konstant ist und ein Zeitverlauf der Leistung des zweiten Pfads konstant ist. Insbesondere kann vorgesehen sein, dass in dem zweiten Betriebszustand die Leistung des ersten Pfads kleiner als die resultierende Leistung ist.

Eine Ausführungsform sieht vor, dass in dem zweiten Betriebszustand des Computertomographiegeräts ein Zeitverlauf der Leistung des ersten Röntgenstrahlers konstant ist und ein Zeitverlauf der Leistung des zweiten Röntgenstrahlers konstant ist. Scans, die mit hoher Leistung ohne Dosismodulation durchgeführt werden, beispielsweise Cardio-Scans, dauern meist weniger als 100 Millisekunden.

Eine Ausführungsform sieht vor, dass in dem zweiten Betriebszustand des Computertomographiegeräts die Leistung des ersten Pfads im Wesentlichen gleich der Leistung des zweiten Pfads ist und/oder dass in dem zweiten Betriebszustand des Computertomographiegeräts die Leistung des ersten Röntgenstrahlers im Wesentlichen gleich der Leistung des zweiten Röntgenstrahlers ist.

Beispielsweise kann vorgesehen sein, dass in dem zweiten Betriebszustand des Computertomographiegeräts die Leistung des zweiten Pfads im Wesentlichen gleich der Leistung des ersten Pfads ist, beispielsweise größer als 80% und kleiner als 120% der Leistung des ersten Pfads ist, insbesondere größer als 90% und kleiner als 110% der Leistung des ersten Pfads ist.

Beispielsweise kann in dem zweiten Betriebszustand des Computertomographiegeräts die Leistung des ersten Pfads größer als 90 Kilowatt, insbesondere größer als 100 Kilowatt, und/oder kleiner als 150 Kilowatt, insbesondere kleiner als 140 Kilowatt, sein. Beispielsweise kann in dem zweiten Betriebszustand des Computertomographiegeräts die Leistung des ersten Pfads größer als 110 Kilowatt und/oder kleiner als 130 Kilowatt, beispielsweise gleich 120 Kilowatt sein.

Beispielsweise kann in dem zweiten Betriebszustand des Computertomographiegeräts die Leistung des zweiten Pfads größer als 90 Kilowatt, insbesondere größer als 100 Kilowatt, und/oder kleiner als 150 Kilowatt, insbesondere kleiner als 140 Kilowatt, sein. Beispielsweise kann in dem zweiten Betriebszustand des Computertomographiegeräts die Leistung des zweiten Pfads größer als 110 Kilowatt und/oder kleiner als 130 Kilowatt, beispielsweise gleich 120 Kilowatt sein.

Dem zweiten Betriebszustand des Computertomographiegeräts kann beispielsweise ein Ladezustand des Computertomographiegeräts vorausgehen, in dem die elektrische Energie aus dem Niederspannungsnetz in den Energiespeicher geladen wird. Die Dauer des Ladezustands kann beispielsweise mehrere Sekunden umfassen. Die Dauer des zweiten Betriebszustands des Computertomographiegeräts kann beispielsweise größer als 50 Millisekunden, insbesondere größer als 80 Millisekunden, und/oder kleiner als 150 Millisekunden, insbesondere kleiner als 120 Millisekunden sein. Die Dauer des zweiten Betriebszustands des Computertomographiegeräts kann beispielsweise größer als 90 Millisekunden und/oder kleiner als 110 Millisekunden, beispielsweise gleich 100 Millisekunden sein.

Eine Ausführungsform sieht vor, dass das Computertomographiegerät eine Steuerungsvorrichtung aufweist, welche zum Einstellen des ersten Betriebszustands des Computertomographiegeräts und/oder zum Einstellen des zweiten Betriebszustands des Computertomographiegeräts ausgebildet ist. Insbesondere kann die Steuerungsvorrichtung mit dem Eingangsmodul und mit dem Ausgangsmodul mittels einer Steuerungsdatenübertragungsverbindung zum Übertragen von Steuerungsdaten verbunden sein.

Eine Ausführungsform sieht vor, dass der Energiespeicher eine Mehrzahl von Superkondensatorzellen aufweist, welche die elektrische Kapazität und die elektrochemische Pseudokapazität bilden, auf welchen der Energiespeicher basiert.

Die Mehrzahl von Superkondensatorzellen kann beispielsweise aus mehr als 100, insbesondere mehr als 200, Superkondensatorzellen und/oder weniger als 400, insbesondere weniger als 300, Superkondensatorzellen bestehen. Die Mehrzahl von Superkondensatorzellen kann beispielsweise aus 250 Superkondensatorzellen bestehen.

Eine Ausführungsform sieht vor, dass für jede Superkondensatorzelle der Mehrzahl von Superkondensatorzellen die Kapazität der jeweiligen Superkondensatorzelle größer als 10 Farad und/oder kleiner als 20 Farad ist, beispielsweise gleich 15 Farad, ist. Superkondensatoren sind dem Fachmann auch unter den Begriffen elektrochemischer Kondensator, Ultrakondensator, Ultracap und Supercap bekannt. Die elektrische Kapazität des Superkondensators kann insbesondere eine Doppelschichtkapazität sein.

Eine Ausführungsform sieht vor, dass die Masse der Mehrzahl von Superkondensatorzellen insgesamt kleiner als 20 Kilogramm, beispielsweise kleiner als 10 Kilogramm, insbesondere kleiner als 5 Kilogramm, ist. Eine Ausführungsform sieht vor, dass die Masse des Energiespeichers kleiner als 20 Kilogramm, beispielsweise kleiner als 10 Kilogramm, insbesondere kleiner als 5 Kilogramm, ist. Ein solcher Energiespeicher kann beispielsweise mit einem relativ geringen technischen Aufwand und mit geringen Kosten auf dem Rotor des Computertomographiegeräts montiert werden.

Die Erfindung betrifft ferner ein Verfahren zur Leistungsversorgung eines Röntgensystems eines Computertomographiegeräts, das Verfahren umfassend:
- eine Entnahme einer Leistung eines ersten Pfads aus einem Niederspannungsnetz mittels des ersten Pfads und ein Einspeisen der Leistung des ersten Pfads in einen Zwischenkreis, der auf Gleichstrom basiert, mittels des ersten Pfads, wobei der erste Pfad einen Gleichrichter zur Verbindung des Zwischenkreises mit dem Niederspannungsnetz aufweist,
- eine Entnahme von elektrischer Energie aus dem Niederspannungsnetz mittels eines zweiten Pfads und ein Einspeisen einer Leistung des zweiten Pfads in den Zwischenkreis basierend auf der elektrischen Energie mittels des zweiten Pfads, wobei der zweite Pfad einen Energiespeicher zum Speichern der elektrischen Energie, der auf einer elektrischen Kapazität und/oder auf einer elektrochemischen Pseudokapazität basiert, ein Eingangsmodul zur Verbindung des Energiespeichers mit dem Niederspannungsnetz und ein Ausgangsmodul zur Verbindung des Energiespeichers mit dem Zwischenkreis aufweist,
- eine Entnahme einer resultierenden Leistung aus dem Zwischenkreis mittels des Röntgensystems des Computertomographiegeräts, wobei durch die Leistung des zweiten Pfads eine Differenz zwischen der resultierenden Leistung und der Leistung des ersten Pfads ausgeglichen wird.

Eine Ausführungsform sieht vor, dass die elektrische Energie mittels des Energiespeichers gespeichert wird, insbesondere nach der Entnahme der elektrischen Energie aus dem Niederspannungsnetz und/oder vor dem Einspeisen der Leistung des zweiten Pfads in den Zwischenkreis basierend auf der elektrischen Energie mittels des Energiespeichers gespeichert wird.

Erfindungsgemäß ist vorgesehen,
- dass das Röntgensystem einen erster Röntgenstrahler und einen zweiter Röntgenstrahler aufweist,
- wobei mittels des ersten Röntgenstrahlers eine Leistung des ersten Röntgenstrahlers aus dem Zwischenkreis entnommen wird,
- dass mittels des zweiten Röntgenstrahlers eine Leistung des zweiten Röntgenstrahlers aus dem Zwischenkreis entnommen wird, und
- dass die resultierende Leistung aus der Leistung des ersten Röntgenstrahlers und der Leistung des zweiten Röntgenstrahlers resultiert.

Das Verfahren umfasst ein Betreiben des Computertomographiegeräts in einem ersten Betriebszustand des Computertomographiegeräts, in dem ein Zeitverlauf der resultierenden Leistung periodisch ist, ein Zeitverlauf der Leistung des ersten Pfads konstant ist und ein Zeitverlauf der Leistung des zweiten Pfads periodisch ist.

Erfindungsgemäß ist vorgesehen, dass mittels des zweiten Pfads in dem ersten Betriebszustand des Computertomographiegeräts die elektrische Energie aus dem Wechselstromkreis in den Energiespeicher geladen wird, während der Zeitverlauf der resultierenden Leistung einen minimalen Wert der resultierenden Leistung durchläuft, und die Leistung des zweiten Pfads basierend auf der elektrischen Energie aus dem Energiespeicher in den Zwischenkreis eingespeist wird, während der Zeitverlauf der resultierenden Leistung den maximalen Wert der resultierenden Leistung durchläuft.

Erfindungsgemäß ist vorgesehen,
- dass das Röntgensystem um eine Rotationsachse, um die es drehbar gelagert ist, gedreht wird,
- dass der erste Röntgenstrahler und der zweite Röntgenstrahler in einer Rotationsebene, die zu der Rotationsachse senkrecht ist, um einen Systemwinkel, dessen Scheitelpunkt auf der Rotationsachse liegt, zueinander versetzt angeordnet sind,
- dass in dem ersten Betriebszustand des Computertomographiegeräts ein Zeitverlauf der Leistung des ersten Röntgenstrahlers periodisch ist und ein Zeitverlauf der Leistung des zweiten Röntgenstrahlers periodisch ist, wobei eine Periodendauer des Zeitverlaufs der Leistung des ersten Röntgenstrahlers gleich einer Periodendauer eines Zeitverlaufs der Leistung des zweiten Röntgenstrahlers ist, und
- dass eine Phasenverschiebung zwischen dem Zeitverlauf der Leistung des ersten Röntgenstrahlers und dem Zeitverlauf der Leistung des zweiten Röntgenstrahlers dem Systemwinkel entspricht.

Das Verfahren kann ferner ein Betreiben des Computertomographiegeräts in einem zweiten Betriebszustand umfassen, in dem ein Zeitverlauf der resultierenden Leistung konstant ist, ein Zeitverlauf der Leistung des ersten Pfads konstant ist und ein Zeitverlauf der Leistung des zweiten Pfads konstant ist.

Eine Ausführungsform sieht vor,
- dass in dem zweiten Betriebszustand des Computertomographiegeräts die Leistung des ersten Pfads im Wesentlichen gleich der Leistung des zweiten Pfads ist und/oder
- dass in dem zweiten Betriebszustand des Computertomographiegeräts die Leistung des ersten Röntgenstrahlers im Wesentlichen gleich der Leistung des zweiten Röntgenstrahlers ist.

Auf diese Weise können die Spitzeneingangsleistungen, die dem Niederspannungsnetz entnommen werden, und damit die Anforderungen an den Netzanschluss reduziert werden.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, der durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Beispielsweise weist die medizinische Bildgebungsvorrichtung die medizinische Bildgebungsvorrichtung auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind. Ordnungs zahlwort

Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.

Es zeigen:
Fig. 1 jeweils einen beispielhaften Zeitverlauf für die Leistung des ersten Röntgenstrahlers, die Leistung des zweiten Röntgenstrahlers und die resultierende Leistung,
Fig. 2 eine Darstellung des ersten Pfads und des zweiten Pfads,
Fig. 3 ein Computertomographiegerät und
Fig. 4 ein Ablaufdiagramm eines Verfahrens zur Leistungsversorgung eines Röntgensystems eines Computertomographiegeräts.

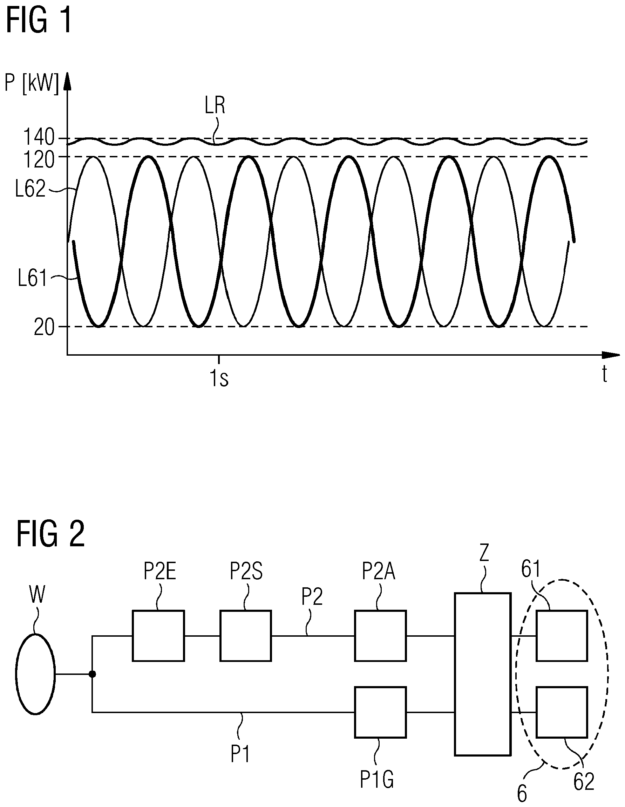

Die Fig. 1 zeigt beispielhaft einen Zeitverlauf L61 einer Leistung des ersten Röntgenstrahlers 61, einen Zeitverlauf L62 einer Leistung des zweiten Röntgenstrahlers 62 und einen Zeitverlauf LR einer resultierenden Leistung. Entlang der Achse P sind Werte in Kilowatt (kW) eingetragen. Entlang der Achse t sind Werte in Sekunden (s) eingetragen.

Jeder der dargestellten Zeitverläufe L61, L62 und LR ist kontinuierlich oszillierend. Die Zeitverläufe L61 und L62 sind sinusförmig. Die resultierende Leistung ist in dem dargestellten Fall die Summe der Leistung des ersten Röntgenstrahlers 61 und der Leistung des zweiten Röntgenstrahlers 62. Bis auf eine Phasenverschiebung zwischen dem Zeitverlauf L61 und dem Zeitverlauf L62, die dem Systemwinkel Y entspricht, sind die Zeitverläufe L61 und L62 identisch.

Fig. 2 zeigt eine Darstellung des ersten Pfads P1 und des zweiten Pfads P2.

Der erste Pfad P1 ist zur Entnahme einer Leistung des ersten Pfads P1 aus dem Niederspannungsnetz W und zum Einspeisen der Leistung des ersten Pfads P1 in den Zwischenkreis Z, der auf Gleichstrom basiert, ausgebildet, wobei der erste Pfad P1 einen Gleichrichter P1G zur Verbindung des Zwischenkreises Z mit dem Niederspannungsnetz W aufweist.

Der zweite Pfad P2 ist zur Entnahme einer elektrischen Energie aus dem Niederspannungsnetz W und zum Einspeisen einer Leistung des zweiten Pfads P2 in den Zwischenkreis Z basierend auf der elektrischen Energie ausgebildet, um durch die Leistung des zweiten Pfads P2 eine Differenz zwischen der resultierenden Leistung und der Leistung des ersten Pfads P1 auszugleichen, wobei der zweite Pfad P2 einen Energiespeicher P2S zum Speichern der Energie, der auf einer elektrischen Kapazität und/oder auf einer elektrochemischen Pseudokapazität basiert, ein Eingangsmodul P2E zur Verbindung des Energiespeichers P2S mit dem Niederspannungsnetz W und ein Ausgangsmodul P2A zur Verbindung des Energiespeichers P2S mit dem Zwischenkreis Z aufweist.

Das Röntgensystem 6 weist den ersten Röntgenstrahler 61 und den zweiten Röntgenstrahlen 62 auf und ist zur Entnahme der resultierenden Leistung aus dem Zwischenkreis Z ausgebildet.

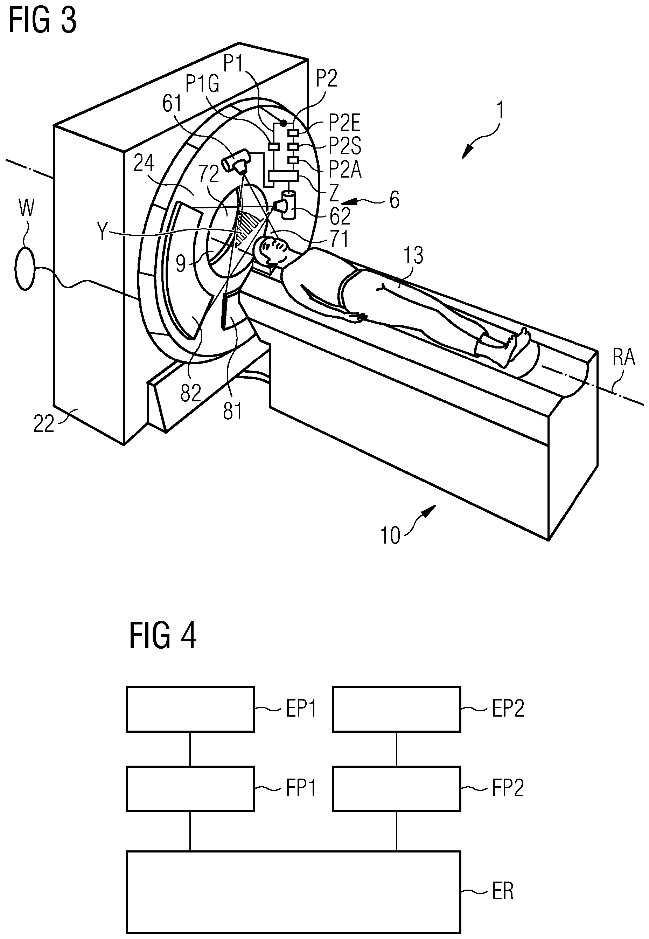

Fig. 3 zeigt ein Computertomographiegerät 1, aufweisend den Tragrahmen 22 und den Rotor 24, der an dem Tragrahmen 22 relativ zu dem Tragrahmen 22 um die Rotationsachse RA drehbar gelagert ist. Der Rotor 24 weist das Röntgensystem 6, insbesondere den ersten Röntgenstrahler 61 und den zweiten Röntgenstrahler 62, auf. Das Röntgensystem 6 ist somit ebenfalls um die Rotationsachse RA relativ zu dem Tragrahmen 22 drehbar gelagert.

Der erste Pfad P1 und der zweite Pfad P2 weisen einen gemeinsamen Abschnitt auf, der unmittelbar an das Niederspannungsnetz W anschließt und eine Drehübertragungsvorrichtung zur Drehübertragung der Leistung des ersten Pfads P1 und der elektrischen Energie des zweiten Pfads P2 von dem Tragrahmen 22 auf den Rotor 24 aufweist. In dem gezeigten gemeinsamen Abschnitt werden die Leistung des ersten Pfads P1 und die elektrische Energie des zweiten Pfads P2 gleichzeitig mittels desselben elektrischen Leiters übertragen.

Der erste Röntgenstrahler 61 ist zum Erzeugen einer ersten Röntgenstrahlung 71, die auf einen ersten Detektor 81 des Computertomographiegeräts 1 ausgerichtet ist, basierend auf der Leistung des ersten Röntgenstrahlers 61 ausgebildet. Der zweite Röntgenstrahler 62 ist zum Erzeugen einer zweiten Röntgenstrahlung 72, die auf den zweiten Detektor 82 des Computertomographiegeräts 1 ausgerichtet ist, basierend auf der Leistung des zweiten Röntgenstrahlers 62 ausgebildet. Der Patient 13 ist mittels der Patientenlagerungsvorrichtung 10 derart entlang einer Längsrichtung des Patienten 13 bewegbar gelagert, dass der Patient 13 entlang der Längsrichtung des Patienten 13 in die tunnelförmige Öffnung 9 des Computertomographiegeräts 1 eingeführt werden kann. Der Systemwinkel Y beträgt ungefähr 95 Grad.

Fig. 4 zeigt ein Ablaufdiagramm eines Verfahrens zur Leistungsversorgung des Röntgensystems 6 eines Computertomographiegeräts 1, das Verfahren umfassend:
- eine Entnahme EP1 einer Leistung eines ersten Pfads P1 aus einem Niederspannungsnetz W mittels des ersten Pfads P1 und ein Einspeisen FP1 der Leistung des ersten Pfads P1 in einen Zwischenkreis Z, der auf Gleichstrom basiert, mittels des ersten Pfads, wobei der erste Pfad P1 einen Gleichrichter P1G zur Verbindung des Zwischenkreises Z mit dem Niederspannungsnetz W aufweist,
- eine Entnahme EP2 von elektrischer Energie aus dem Niederspannungsnetz W mittels eines zweiten Pfads P2 und ein Einspeisen FP2 einer Leistung des zweiten Pfads P2 in den Zwischenkreis Z basierend auf der elektrischen Energie mittels des zweiten Pfads P2, wobei der zweite Pfad P2 einen Energiespeicher P2S zum Speichern der elektrischen Energie, der auf einer elektrischen Kapazität und/oder auf einer elektrochemischen Pseudokapazität basiert, ein Eingangsmodul P2E zur Verbindung des Energiespeichers P2S mit dem Niederspannungsnetz W und ein Ausgangsmodul P2A zur Verbindung des Energiespeichers P2S mit dem Zwischenkreis Z aufweist,
- eine Entnahme ER einer resultierenden Leistung aus dem Zwischenkreis Z mittels des Röntgensystems 6 des Computertomographiegeräts 1, wobei durch die Leistung des zweiten Pfads P2 eine Differenz zwischen der resultierenden Leistung und der Leistung des ersten Pfads P1 ausgeglichen wird.

## Patentansprüche

1. Computertomographiegerät (1), aufweisend
- ein Röntgensystem (6) und einen Zwischenkreis (Z), der auf Gleichstrom basiert, wobei das Röntgensystem (6) zur Entnahme einer resultierenden Leistung aus dem Zwischenkreis (Z) ausgebildet ist,
- einen ersten Pfad (P1), der zur Entnahme einer Leistung des ersten Pfads (P1) aus einem Niederspannungsnetz (W) und zum Einspeisen der Leistung des ersten Pfads (P1) in den Zwischenkreis (Z) ausgebildet ist, wobei der erste Pfad (P1) einen Gleichrichter (P1G) zur Verbindung des Zwischenkreises (Z) mit dem Niederspannungsnetz (W) aufweist,
- einen zweiten Pfad (P2), der zur Entnahme einer elektrischen Energie aus dem Niederspannungsnetz (W) und zum Einspeisen einer Leistung des zweiten Pfads (P2) in den Zwischenkreis (Z) basierend auf der elektrischen Energie ausgebildet ist, um durch die Leistung des zweiten Pfads (P2) eine Differenz zwischen der resultierenden Leistung und der Leistung des ersten Pfads (P1) auszugleichen, wobei der zweite Pfad (P2) einen Energiespeicher (P2S) zum Speichern der Energie, der auf einer elektrischen Kapazität und/oder auf einer elektrochemischen Pseudokapazität basiert, ein Eingangsmodul (P2E) zur Verbindung des Energiespeichers (P2S) mit dem Niederspannungsnetz (W) und ein Ausgangsmodul (P2A) zur Verbindung des Energiespeichers (P2S) mit dem Zwischenkreis (Z) aufweist,
- wobei das Röntgensystem (6) einen ersten Röntgenstrahler (61) und einen zweiten Röntgenstrahler (62) aufweist,
- wobei der erste Röntgenstrahler (61) zur Entnahme einer Leistung des ersten Röntgenstrahlers (61) aus dem Zwischenkreis (Z) ausgebildet ist,
- wobei der zweite Röntgenstrahler (62) zur Entnahme einer Leistung des zweiten Röntgenstrahlers (62) aus dem Zwischenkreis (Z) ausgebildet ist,
- wobei die resultierende Leistung aus der Leistung des ersten Röntgenstrahlers (61) und der Leistung des zweiten Röntgenstrahlers (62) resultiert,
- wobei das Röntgensystem (6) um eine Rotationsachse drehbar gelagert ist,
- wobei der erste Röntgenstrahler (61) und der zweite Röntgenstrahler (62) in einer Rotationsebene, die zu der Rotationsachse senkrecht ist, um einen Systemwinkel (Y), dessen Scheitelpunkt auf der Rotationsachse liegt, zueinander versetzt angeordnet sind,
- wobei in einem ersten Betriebszustand des Computertomographiegeräts (1) ein Zeitverlauf (L61) der Leistung des ersten Röntgenstrahlers (61) periodisch ist und ein Zeitverlauf (L62) der Leistung des zweiten Röntgenstrahlers (62) periodisch ist, wobei eine Periodendauer des Zeitverlaufs der Leistung des ersten Röntgenstrahlers (61) gleich einer Periodendauer eines Zeitverlaufs der Leistung des zweiten Röntgenstrahlers (62) ist,
- wobei eine Phasenverschiebung zwischen dem Zeitverlauf (L61) der Leistung des ersten Röntgenstrahlers (61) und dem Zeitverlauf (L62) der Leistung des zweiten Röntgenstrahlers (62) dem Systemwinkel (Y) entspricht,
- **dadurch gekennzeichnet, dass** in einem ersten Betriebszustand des Computertomographiegeräts (1) ein Zeitverlauf (LR) der resultierenden Leistung periodisch ist, ein Zeitverlauf der Leistung des ersten Pfads (P1) konstant ist und ein Zeitverlauf der Leistung des zweiten Pfads (P2) periodisch ist, wobei der zweite Pfad derart ausgebildet ist, dass in dem ersten Betriebszustand des Computertomographiegeräts (1) die elektrische Energie aus dem Niederspannungsnetz in den Energiespeicher (P2S) geladen werden kann, während der Zeitverlauf (LR) der resultierenden Leistung einen minimalen Wert der resultierenden Leistung durchläuft, und die Leistung des zweiten Pfads (P2) basierend auf der elektrischen Energie aus dem Energiespeicher (P2S) in den Zwischenkreis (Z) eingespeist werden kann, während der Zeitverlauf (LR) der resultierenden Leistung einen maximalen Wert der resultierenden Leistung durchläuft.

2. Computertomographiegerät (1) nach Anspruch 1,
- wobei in einem zweiten Betriebszustand des Computertomographiegeräts (1) ein Zeitverlauf (LR) der resultierenden Leistung konstant ist, ein Zeitverlauf der Leistung des ersten Pfads (P1) konstant ist und ein Zeitverlauf der Leistung des zweiten Pfads (P2) konstant ist.

3. Computertomographiegerät (1) nach Anspruch 2,
- wobei in dem zweiten Betriebszustand des Computertomographiegeräts (1) die Leistung des ersten Pfads (P1) im Wesentlichen gleich der Leistung des zweiten Pfads (P2) ist und/oder
- wobei in dem zweiten Betriebszustand des Computertomographiegeräts (1) die Leistung des ersten Röntgenstrahlers (61) im Wesentlichen gleich der Leistung des zweiten Röntgenstrahlers (62) ist.

4. Computertomographiegerät (1) nach einem der Ansprüche 1 bis 3,
- wobei der Energiespeicher (P2S) eine Mehrzahl von Superkondensatorzellen aufweist, welche die elektrische Kapazität und die elektrochemische Pseudokapazität bilden, auf welchen der Energiespeicher (P2S) basiert.

5. Verfahren zur Leistungsversorgung eines Röntgensystems (6) eines Computertomographiegeräts (1), das Verfahren umfassend:
- eine Entnahme (EP1) einer Leistung eines ersten Pfads (P1) aus einem Niederspannungsnetz (W) mittels des ersten Pfads (P1) und ein Einspeisen (FP1) der Leistung des ersten Pfads (P1) in einen Zwischenkreis (Z), der auf Gleichstrom basiert, mittels des ersten Pfads, wobei der erste Pfad (P1) einen Gleichrichter (P1G) zur Verbindung des Zwischenkreises (Z) mit dem Niederspannungsnetz (W) aufweist,
- eine Entnahme (EP2) von elektrischer Energie aus dem Niederspannungsnetz (W) mittels eines zweiten Pfads (P2) und ein Einspeisen (FP2) einer Leistung des zweiten Pfads (P2) in den Zwischenkreis (Z) basierend auf der elektrischen Energie mittels des zweiten Pfads (P2), wobei der zweite Pfad (P2) einen Energiespeicher (P2S) zum Speichern der elektrischen Energie, der auf einer elektrischen Kapazität und/oder auf einer elektrochemischen Pseudokapazität basiert, ein Eingangsmodul (P2E) zur Verbindung des Energiespeichers (P2S) mit dem Niederspannungsnetz (W) und ein Ausgangsmodul (P2A) zur Verbindung des Energiespeichers (P2S) mit dem Zwischenkreis (Z) aufweist,
- eine Entnahme (ER) einer resultierenden Leistung aus dem Zwischenkreis (Z) mittels des Röntgensystems (6) des Computertomographiegeräts (1), wobei durch die Leistung des zweiten Pfads (P2) eine Differenz zwischen der resultierenden Leistung und der Leistung des ersten Pfads (P1) ausgeglichen wird,
- wobei das Röntgensystem (6) einen ersten Röntgenstrahler (61) und einen zweiten Röntgenstrahler (62) aufweist,
- wobei mittels des ersten Röntgenstrahlers (61) eine Leistung des ersten Röntgenstrahlers (61) aus dem Zwischenkreis (Z) entnommen wird,
- wobei mittels des zweiten Röntgenstrahlers (62) eine Leistung des zweiten Röntgenstrahlers (62) aus dem Zwischenkreis (Z) entnommen wird,
- wobei die resultierende Leistung aus der Leistung des ersten Röntgenstrahlers (61) und der Leistung des zweiten Röntgenstrahlers (62) resultiert,
- wobei das Röntgensystem (6) um eine Rotationsachse, um die es drehbar gelagert ist, gedreht wird,
- wobei der erste Röntgenstrahler (61) und der zweite Röntgenstrahler (62) in einer Rotationsebene, die zu der Rotationsachse senkrecht ist, um einen Systemwinkel, dessen Scheitelpunkt auf der Rotationsachse liegt, zueinander versetzt angeordnet sind,
- wobei in einem ersten Betriebszustand des Computertomographiegeräts (1) ein Zeitverlauf (L61) der Leistung des ersten Röntgenstrahlers (61) periodisch ist und ein Zeitverlauf (L62) der Leistung des zweiten Röntgenstrahlers (62) periodisch ist, wobei eine Periodendauer des Zeitverlaufs der Leistung des ersten Röntgenstrahlers (61) gleich einer Periodendauer eines Zeitverlaufs der Leistung des zweiten Röntgenstrahlers (62) ist,
- wobei eine Phasenverschiebung zwischen dem Zeitverlauf (L61) der Leistung des ersten Röntgenstrahlers (61) und dem Zeitverlauf (L62) der Leistung des zweiten Röntgenstrahlers (62) dem Systemwinkel (Y) entspricht,
- **gekennzeichnet durch** ein Betreiben des Computertomographiegeräts (1) in einem ersten Betriebszustand des Computertomographiegeräts (1), in dem ein Zeitverlauf (LR) der resultierenden Leistung periodisch ist, ein Zeitverlauf der Leistung des ersten Pfads (P1) konstant ist und ein Zeitverlauf der Leistung des zweiten Pfads (P2) periodisch ist, wobei mittels des zweiten Pfads in dem ersten Betriebszustand des Computertomographiegeräts (1) die elektrische Energie aus dem Niederspannungsnetz in den Energiespeicher (P2S) geladen wird, während der Zeitverlauf (LR) der resultierenden Leistung einen minimalen Wert der resultierenden Leistung durchläuft, und die Leistung des zweiten Pfads (P2) basierend auf der elektrischen Energie aus dem Energiespeicher (P2S) in den Zwischenkreis (Z) eingespeist wird, während der Zeitverlauf (LR) der resultierenden Leistung einen maximalen Wert der resultierenden Leistung durchläuft.

6. Verfahren nach Anspruch 5, ferner umfassend
- ein Betreiben des Computertomographiegeräts (1) in einem zweiten Betriebszustand, in dem ein Zeitverlauf (LR) der resultierenden Leistung konstant ist, ein Zeitverlauf der Leistung des ersten Pfads (P1) konstant ist und ein Zeitverlauf der Leistung des zweiten Pfads (P2) konstant ist.

7. Verfahren nach Anspruch 6,
- wobei in dem zweiten Betriebszustand des Computertomographiegeräts (1) die Leistung des ersten Pfads (P1) im Wesentlichen gleich der Leistung des zweiten Pfads (P2) ist und/oder
- wobei in dem zweiten Betriebszustand des Computertomographiegeräts (1) die Leistung des ersten Röntgenstrahlers (61) im Wesentlichen gleich der Leistung des zweiten Röntgenstrahlers (62) ist.

## Claims

1. Computed tomography device (1), having
- an x-ray system (6) and an intermediate circuit (Z), which is based on direct current, wherein the x-ray system (6) is embodied to take a resulting power from the intermediate circuit (Z),
- a first path (P1), which is embodied to take a power of the first path (P1) from a low voltage network (W) and to feed the power of the first path (P1) into the intermediate circuit (Z), wherein the first path (P1) has a rectifier (P1G) for connecting the intermediate circuit (Z) to the low voltage network (W),
- a second path (P2), which is embodied to take an electrical energy from the low voltage network (W) and to feed a power of the second path (P2) into the intermediate circuit (Z) on the basis of the electrical energy, in order to balance out a difference between the resulting power and the power of the first path (P1) by means of the power of the second path (P2), wherein the second path (P2) has an energy storage device (P2S) for storing the energy, which is based on an electrical capacitance and/or on an electrochemical pseudo capacitance, an input module (P2E) for connecting the energy storage device (P2S) to the low voltage network (W) and an output module (P2A) for connecting the energy storage device (P2S) to the intermediate circuit (Z),
- wherein the x-ray system (6) has a first x-ray emitter (61) and a second x-ray emitter (62),
- wherein the first x-ray emitter (61) is embodied to take a power of the first x-ray emitter (61) from the intermediate circuit (Z),
- wherein the second x-ray emitter (62) is embodied to take a power of the second x-ray emitter (62) from the intermediate circuit (Z),
- wherein the resulting power results from the power of the first x-ray emitter (61) and the power of the second x-ray emitter (62),
- wherein the x-ray system (6) is rotatably mounted about an axis of rotation,
- wherein the first x-ray emitter (61) and the second x-ray emitter (62) are arranged offset with respect to one another in a rotational plane, which is perpendicular to the axis of rotation, by a system angle (Y), the apex of which lies on the axis of rotation,
- wherein in a first operating state of the computed tomography device (1), a course of time (L61) of the power of the first x-ray emitter (61) is periodic and a course of time (L62) of the power of the second x-ray emitter (62) is periodic, wherein a cycle duration of the course of time of the power of the first x-ray emitter (61) is equal to a cycle duration of a course of time of the power of the second x-ray emitter (62),
- wherein a phase shift between the course of time (L61) of the power of the first x-ray emitter (61) and the course of time (L62) of the power of the second x-ray emitter (62) corresponds to the system angle (Y),
- **characterised in that** in a first operating state of the computed tomography device (1) a course of time (LR) of the resulting power is periodic, a course of time of the power of the first path (P1) is constant, and a course of time of the power of the second path (P2) is periodic, wherein the second path is embodied so that in the first operating state of the computed tomography device (1), the electrical energy can be loaded from the low voltage network into the energy storage device (P2S), while the course of time (LR) of the resulting power runs through a minimal value of the resulting power, and the power of the second path (P2) can be fed into the intermediate circuit (Z) on the basis of the electrical energy from the energy storage device (P2S), while the course of time (LR) of the resulting power runs through a maximum value of the resulting power.

2. Computed tomography device (1) according to claim 1,
- wherein in a second operating state of the computed tomography device (1), a course of time (LR) of the resulting power is constant, a course of time of the power of the first path (P1) is constant and a course of time of the power of the second path (P2) is constant.

3. Computed tomography device (1) according to claim 2,
- wherein in the second operating state of the computed tomography device (1), the power of the first path (P1) is substantially equal to the power of the second path (P2) and/or
- wherein in the second operating state of the computed tomography device (1), the power of the first x-ray emitter (61) is substantially equal to the power of the second x-ray emitter (62).

4. Computed tomography device (1) according to one of claims 1 to 3,
- wherein the energy storage device (P2S) has a plurality of super capacitor cells, which form the electrical capacitance and the electrochemical pseudo capacitance, on which the energy storage device (P2S) is based.

5. Method for supplying power to an x-ray system (6) of a computed tomography device (1), the method comprising:
- taking (EP1) a power of a first path (P1) from a low voltage network (W) by means of the first path (P1) and feeding (FP1) the power of the first path (P1) into an intermediate circuit (Z), which is based on direct current, by means of the first path, wherein the first path (P1) has a rectifier (P1G) for connecting the intermediate circuit (Z) to the low voltage network (W),
- taking (EP2) electrical energy from the low voltage network (W) by means of a second path (P2) and feeding (FP2) a power of the second path (P2) into the intermediate circuit (Z) on the basis of the electrical energy by means of the second path (P2), wherein the second path (P2) has an energy storage device (P2S) for storing the electrical energy, which is based on an electrical capacitance and/or on an electrochemical pseudo capacitance, an input module (P2E) for connecting the energy storage device (P2S) to the low voltage network (W) and an output module (P2A) for connecting the energy storage device (P2S) to the intermediate circuit (Z),
- taking (ER) a resulting power from the intermediate circuit (Z) by means of the x-ray system (6) of the computed tomography device (1), wherein a difference between the resulting power and the power of the first path (P1) is balanced out by the power of the second path (P2),
- wherein the x-ray system (6) has a first x-ray emitter (61) and a second x-ray emitter (62),
- wherein a power of the first x-ray emitter (61) is taken from the intermediate circuit (Z) by means of the first x-ray emitter (61),
- wherein a power of the second x-ray emitter (62) is taken from the intermediate circuit (Z) by means of the second x-ray emitter (62),
- wherein the resulting power results from the power of the first x-ray emitter (61) and the power of the second x-ray emitter (62),
- wherein the x-ray system (6) is rotated about an axis of rotation, about which it is rotatably mounted,
- wherein the first x-ray emitter (61) and the second x-ray emitter (62) are arranged offset with respect to one another in a rotational plane, which is perpendicular to the axis of rotation, by a system angle, the apex of which lies on the axis of rotation,
- wherein in a first operating state of the computed tomography device (1), a course of time (L61) of the power of the first x-ray emitter (61) is periodic and a course of time (L62) of the power of the second x-ray emitter (62) is periodic, wherein a cyclic duration of the course of time of the power of the first x-ray emitter (61) is equal to a cyclic duration of a course of time of the power of the second x-ray emitter (62),
- wherein a phase shift between the course of time (L61) of the power of the first x-ray emitter (61) and the course of time (L62) of the power of the second x-ray emitter (62) corresponds to the system angle (Y),
- **characterised by** an operation of the computed tomography device (1) in a first operating state of the computed tomography device (1), in which a course of time (LR) of the resulting power is periodic, a course of time of the power of the first path (P1) is constant and a course of time of the power of the second path (P2) is periodic, wherein in the first operating state of the computed tomography device (1) the electrical energy is loaded from the low voltage network into the energy storage device (P2S) by means of the second path, while the course of time (LR) of the resulting power runs through a minimal value of the resulting power, and the power of the second path (P2) is fed into the intermediate circuit (Z) on the basis of the electrical energy from the energy storage device (P2S), while the course of time (LR) of the resulting power runs through a maximum value of the resulting power.

6. Method according to claim 5, further comprising
- operating the computed tomography device (1) in a second operating state, in which a course of time (LR) of the resulting power is constant, a course of time of the power of the first path (P1) is constant and a course of time of the power of the second path (P2) is constant.

7. Method according to claim 6,
- wherein in the second operating state of the computed tomography device (1), the power of the first path (P1) is substantially equal to the power of the second path (P2), and/or
- wherein in the second operating state of the computed tomography device (1), the power of the first x-ray emitter (61) is substantially equal to the power of the second x-ray emitter (62).

## Revendications

1. Appareil (1) de tomodensitométrie assisté par ordinateur, comportant
- un système (6) de rayons X et un circuit (Z) intermédiaire, qui repose sur du courant continu, dans lequel le système (6) de rayons X est constitué pour le prélèvement d'une puissance résultante du circuit (Z) intermédiaire,
- un premier chemin (P1), qui est constitué pour prélever une puissance du premier chemin (P1) d'un réseau (W) en basse tension et pour l'injection de la puissance du premier chemin (P1) dans le circuit (Z) intermédiaire, dans lequel le premier chemin (P1) a un redresseur (P1G) de liaison du circuit (Z) intermédiaire au réseau (W) en basse tension,
- un deuxième chemin (P2) qui est constitué pour le prélèvement d'une énergie électrique du réseau (W) en basse tension et pour injecter une puissance du deuxième réseau (P2) dans le circuit (Z) intermédiaire sur la base de l'énergie électrique, afin de compenser par la puissance du deuxième chemin (P2) une différence entre la puissance résultante et la puissance du premier chemin (P1), dans lequel le deuxième chemin (P2) a un accumulateur (P2S) d'énergie pour l'accumulation de l'énergie, qui repose sur une capacité électrique et/ou sur une pseudo capacité électrochimique, un module (P2E) d'entrée de liaison de l'accumulateur (P2S) d'énergie au réseau (W) en basse tension et un module (P2A) de sortie de liaison de l'accumulateur (P2S) d'énergie au circuit (Z) intermédiaire,
- dans lequel le système (6) de rayons X a un premier émetteur (61) de rayons X et un deuxième émetteur (62) de rayons X,
- dans lequel le premier émetteur (61) de rayons X est constitué pour prélever une puissance du premier émetteur (61) de rayons X du circuit (Z) intermédiaire,
- dans lequel le deuxième émetteur (62) de rayons X est constitué pour prélever une puissance du deuxième émetteur (62) de rayons X du circuit (Z) intermédiaire,
- dans lequel la puissance résultante résulte de la puissance du premier émetteur (61) de rayons X et de la puissance du deuxième émetteur (62) de rayons X,
- dans lequel le système (6) de rayons X est monté tournant autour d'un axe de rotation,
- dans lequel le premier émetteur (61) de rayons X et le deuxième émetteur (62) de rayons X sont disposés en étant décalés l'un par rapport à l'autre dans un plan de rotation, qui est perpendiculaire à l'axe de rotation, d'un angle (Y) de système, dont le sommet est sur l'axe de rotation,
- dans lequel, dans un premier état de fonctionnement de l'appareil (1) de tomodensitométrie assisté par ordinateur, une courbe (L61) en fonction du temps de la puissance du premier émetteur (61) de rayons X est périodique et une courbe (L62) en fonction du temps de la puissance du deuxième émetteur (62) de rayons X est périodique, dans lequel une période de la courbe en fonction du temps de la puissance du premier émetteur (61) de rayons X est égale à une période d'une courbe en fonction du temps de la puissance du deuxième émetteur (62) de rayons X,
- dans lequel un déphasage entre la courbe (L61) en fonction du temps de la puissance du premier émetteur (61) de rayons X et la courbe (L62) en fonction du temps de la puissance du deuxième émetteur (62) de rayons X correspond à l'angle (Y) de système, **caractérisé en ce que**, dans un premier état de fonctionnement de l'appareil (1) de tomodensitométrie assisté par ordinateur, une courbe (LR) en fonction du temps de la puissance résultante est périodique, une courbe en fonction du temps de la puissance du premier chemin (P1) est constante et une courbe en fonction du temps de la puissance du deuxième chemin (P2) est périodique, dans lequel le deuxième chemin est constitué de manière à ce que, dans le premier état de fonctionnement de l'appareil (1) de tomodensitométrie assisté par ordinateur, l'énergie électrique puisse être chargée du réseau en basse tension dans l'accumulateur (P2S) d'énergie, tandis que la courbe (LR) en fonction du temps de la puissance résultante passe par une valeur minimum de la puissance résultante et la puissance du deuxième chemin (P2) reposant sur l'énergie électrique de l'accumulateur (P2S) d'énergie peut être injectée dans le circuit (Z) intermédiaire, tandis que la courbe (LR) en fonction du temps de la puissance résultante passe par une valeur maximum de la puissance résultante.

2. Appareil (1) de tomodensitométrie assisté par ordinateur suivant la revendication 1,
- dans lequel, dans un deuxième état de fonctionnement de l'appareil (1) de tomodensitométrie assisté par ordinateur, une courbe (LR) en fonction du temps de la puissance est constante, une courbe en fonction du temps de la puissance du premier chemin (P1) est constante et une courbe en fonction du temps de la puissance du deuxième chemin (P2) est constante.

3. Appareil (1) de tomodensitométrie assisté par ordinateur suivant la revendication 2,
- dans lequel, dans le deuxième état de fonctionnement de l'appareil (1) de tomodensitométrie assisté par ordinateur, la puissance du premier chemin (P1) est sensiblement égale à la puissance du deuxième chemin (P2) et/ou
- dans lequel, dans le deuxième état de fonctionnement de l'appareil (1) de tomodensitométrie assisté par ordinateur, la puissance du premier émetteur (61) de rayons X est sensiblement égale à la puissance du deuxième émetteur (62) de rayons X.

4. Appareil (1) de tomodensitométrie assisté par ordinateur suivant l'une des revendications 1 à 3,
- dans lequel l'accumulateur (P2S) d'énergie a une pluralité de cellules de super condensateur, qui forment la capacité électrique et la pseudo capacité électrochimique, sur lesquelles repose l'accumulateur (P2S) d'énergie.

5. Procédé d'alimentation en puissance d'un système (6) de rayons X d'un appareil (1) de tomodensitométrie assisté par ordinateur, le procédé comprenant :
- un prélèvement (EP1) d'une puissance d'un premier chemin (P1) d'un réseau (W) en basse tension au moyen du premier chemin (P1) et une injection (FP1) de la puissance du premier chemin (P1) dans un circuit (Z) intermédiaire, qui repose sur du courant continu, au moyen du premier chemin, dans lequel le premier chemin (P1) a un redresseur (P1G) de liaison du circuit (Z) intermédiaire au réseau (W) en basse tension,
- un prélèvement (EP2) d'énergie électrique du réseau (W) en basse tension au moyen d'un deuxième chemin (P2) et une injection (FP2) d'une puissance du deuxième chemin (P2) dans le circuit (Z) intermédiaire sur la base de l'énergie électrique au moyen du deuxième chemin (P2), dans lequel le deuxième chemin (P2) a un accumulateur (P2S) d'énergie pour accumuler l'énergie électrique, qui repose sur une capacité électrique et/ou une pseudo capacité électrochimique, un module (P2E) d'entrée de liaison de l'accumulateur (P2S) d'énergie au réseau (W) en basse tension et un module (P2A) de sortie de liaison de l'accumulateur (P2S) d'énergie au circuit (Z) intermédiaire,
- un prélèvement (ER) d'une puissance résultante du circuit (Z) intermédiaire au moyen du système (6) de rayons X de l'appareil (1) de tomodensitométrie assisté par ordinateur, dans lequel par la puissance du deuxième chemin (P2) on compense une différence entre la puissance résultante et la puissance du premier chemin (P1),
- dans lequel le système (6) de rayons X a un premier émetteur (61) de rayons X et un deuxième émetteur (62) de rayons X,
- dans lequel au moyen du premier émetteur (61) de rayons X, on prélève une puissance du premier émetteur (61) de rayons X du circuit (Z) intermédiaire,
- dans lequel au moyen du deuxième émetteur (62) de rayons X, on prélève une puissance du deuxième émetteur (62) de rayons X du circuit (Z) intermédiaire,
- dans lequel la puissance résultante résulte de la puissance du premier émetteur (61) de rayons X et de la puissance du deuxième émetteur (62) de rayons X,
- dans lequel on fait tourner le système (6) de rayons X autour d'un axe de rotation, autour duquel il est monté tournant,
- dans lequel le premier émetteur (61) de rayons X et le deuxième émetteur (62) de rayons X sont disposés décalés l'un par rapport à l'autre, dans un plan de rotation perpendiculaire à l'axe de rotation, d'un angle de système dont le point sommet se trouve sur l'axe de rotation,
- dans lequel, dans un premier état de fonctionnement de l'appareil (1) de tomodensitométrie assisté par ordinateur, une courbe (L61) en fonction du temps de la puissance du premier émetteur (61) de rayons X est périodique et une courbe (L62) en fonction du temps de la puissance du deuxième émetteur (62) de rayons X est périodique, dans lequel une période de la courbe en fonction du temps de la puissance du premier émetteur (61) de rayons X est égale à une période d'une courbe en fonction du temps de la puissance du deuxième émetteur (62) de rayons X,
- dans lequel un déphasage entre la courbe (L61) en fonction du temps de la puissance du premier émetteur (61) de rayons X et la courbe (L62) en fonction du temps de la puissance du deuxième émetteur (62) de rayons X correspond à l'angle (Y) de système,
- **caractérisé par** un fonctionnement de l'appareil (1) de tomodensitométrie assisté par ordinateur dans un premier état de fonctionnement de l'appareil (1) de tomodensitométrie assisté par ordinateur, dans lequel une courbe (LR) en fonction du temps de la puissance résultante est périodique, une courbe en fonction du temps de la puissance du premier chemin (P1) est constante et une courbe en fonction du temps de la puissance du deuxième chemin (P2) est périodique, dans lequel, au moyen du deuxième chemin dans le premier état de fonctionnement de l'appareil (1) de tomodensitométrie assisté par ordinateur, on charge l'énergie électrique du réseau en basse tension dans l'accumulateur (P2S) d'énergie, tandis que la courbe (LR) en fonction du temps de la puissance résultante passe par une valeur minimum de la puissance résultante, et on injecte la puissance du deuxième chemin (P2) reposant sur l'énergie électrique de l'accumulateur (P2S) d'énergie dans le circuit (Z) intermédiaire, tandis que la courbe (LR) en fonction du temps de la puissance résultante passe par une valeur maximum de la puissance résultante.

6. Procédé suivant la revendication 5, comprenant en outre
- un fonctionnement de l'appareil (1) de tomodensitométrie assisté par ordinateur dans un deuxième état de fonctionnement, dans lequel une courbe (LR) en fonction du temps de la puissance résultante est constante, une courbe en fonction du temps de la puissance du premier chemin (P1) est constante et une courbe en fonction du temps de la puissance du deuxième chemin (P2) est constante.

7. Procédé suivant la revendication 6,
- dans lequel dans le deuxième état de fonctionnement de l'appareil (1) de tomodensitométrie assisté par ordinateur, la puissance du premier chemin (P1) est sensiblement égale à la puissance du deuxième chemin (P2)
et/ou
- dans lequel dans un deuxième état de fonctionnement de l'appareil (1) de tomodensitométrie assisté par ordinateur, la puissance du premier émetteur (61) de rayons X est sensiblement égale à la puissance du deuxième émetteur (62) de rayons X.
